# EUROPEAN PATENT APPLICATION

(11) **EP 3 858 884 A1**
(43) Date of publication of application: **04.08.2021**
(21) Application number: 21161865.7
(22) Date of filing: 28.03.2017
(51) Int. Cl.: C08G 18/66, C08G 18/72, C08G 18/08, C08G 18/76, A61C 1/00, B29C 64/00, B33Y 70/00, C09D 11/00

(54) **THERMOPLASTIC POLYURETHANE COMPOSITIONS FOR SOLID FREEFORM FABRICATION OF ORAL CARE AND MEDICAL DEVICES AND COMPONENTS**

(30) Priority: 31.03.2016 US 201662315905 P
(62) Divisional of application: 17717033.9
(71) Applicant: Lubrizol Advanced Materials, Inc., Cleveland, OH 44141-3247 (US)
(72) Inventor: GREEN, Jennifer, Cleveland, Ohio 44141-3247 (US); COX, John M., Cleveland, Ohio 44141-3247 (US); VONTORCIK, Joseph J. Jr., Cleveland, Ohio 44141-3247 (US); MORGAN, Barbara, Cleveland, Ohio 44141-3247 (US)
(74) Representative: Bradley, Josephine Mary

(57) **Abstract**

The invention relates to compositions and methods for solid freeform fabrication of oral care or medical devices, components and applications, in which the composition includes a thermoplastic polyurethane which is particularly suited for such processing. The useful thermoplastic polyurethanes are derived from (a) an aromatic diisocyanate component, (b) a chain extender component, and (c) a polyol component.

## Description

### FIELD OF THE INVENTION

The invention relates to compositions and methods for the direct solid freeform fabrication of oral care devices and medical, components and applications. The oral care and medical devices can be formed from biocompatible thermoplastic polyurethanes suited for such processing. The useful thermoplastic polyurethanes are derived from (a) an aromatic diisocyanate component, (b) a chain extender component, and (c) an optional polyol component.

### BACKGROUND

Solid Freeform Fabrication (SFF), also referred to as additive manufacturing, is a technology enabling fabrication of arbitrarily shaped structures directly from computer data via additive formation steps. The basic operation of any SFF system consists of slicing a three-dimensional computer model into thin cross sections, translating the result into two-dimensional position data and feeding the data to control equipment which fabricates a three-dimensional structure in a layerwise manner.

Solid freeform fabrication entails many different approaches, including three-dimensional printing, electron beam melting, stereolithography, selective laser sintering, laminated object manufacturing, fused deposition modeling and others.

The differences between these processes lies in the way the layers are placed to create parts, as well as in the materials utilized. Some methods, such as selective laser sintering (SLS), fused deposition modeling (FDM) or fused filament fabrication (FFF), melt or soften the material to produce the layers. Other methods, such as stereolithography (SLA), cure liquid materials.

Typically, additive manufacturing for thermoplastics utilizes two types of printing methods. In the first method, known as an extrusion type, a filament and/or a resin (referred to as "pellet printing") of the subject material is softened or melted then deposited by the machine in layers to form the desired object. Extrusion type methods are known as fused deposition modeling (FDM) or fused filament fabrication (FFF). In extrusion methods, a thermoplastic resin or a strand of thermoplastic filament is supplied to a nozzle head which heats the thermoplastic and turns the flow on and off. The part is constructed by extruding small beads of material which harden to form layers.

The second method is the powder or granular type where a powder is deposited in a granular bed and then fused to the previous layer by selective fusing or melting. The technique typically fuses parts of the layer using a high powered laser. After each cross-section is processed, the powder bed is lowered. A new layer of powdered material is then applied and the steps are repeated until the part is fully constructed. Often, the machine is designed with the capability to preheat the bulk powder bed material to slightly below its melting point. This reduces the amount of energy and time for the laser to increase the temperature of the selected regions to the melting point.

Unlike extrusion methods, the granular or powder methods use the unfused media to support projections or ledges and thin walls in the part being produced. This reduces or eliminates the need for temporary supports as the piece is being constructed. Specific methods include selective laser sintering (SLS), selective heat sintering (SHS) and selective laser melting (SLM). In SLM, the laser completely melts the powder. This allows the formation of a part in a layer-wise method that will have the mechanical properties similar to those of conventionally manufactured parts. Another powder or granular method utilizes an inkjet printing system. In this technique, the piece is created layer-wise by printing a binder in the cross-section of the part using an inkjet-like process on top of a layer of powder. An additional layer of powder is added and the process is repeated until each layer has been printed.

Current solid freeform fabrication for oral care devices and applications has been focused on indirect fabrication, such as printing of molds which are subsequently filled with a material or the printing of a form over which a thermoformed device is then molded; or for medical applications involving visualization, demonstration and mechanical prototyping, e.g., where expected outcomes can be modeled prior to performing procedures based on a 3D-printed prototype. Thus, SFF facilitates rapid fabrication of functioning prototypes with minimal investment in tooling and labor. Such rapid prototyping shortens the product development cycle and improves the design process by providing rapid and effective feedback to the designer. SFF can also be used for rapid fabrication of non-functional parts, e.g., models and the like, for the purpose of assessing various aspects of a design such as aesthetics, fit, assembly and the like.

Current materials utilized in additive manufacturing for oral care and medical applications typically include ABS, nylon, polycarbonates, polycaprolactone, polylactic acid (PLA), poly-L-lactic acid (PLLA) and photopolymers/cured liquid materials. Some of these materials are limited to applications outside the body, such as prototypes, molds, surgical planning and anatomical models, owing to their lack of biocompatibility or long term biodurability.

Given the attractive combination of properties thermoplastic polyurethanes offer, and the wide variety of articles made using more conventional means of fabrication, it would be desirable to identify and/or develop thermoplastic polyurethanes well suited for direct solid freeform fabrication of oral care and medical devices, components and applications.

### SUMMARY

The disclosed technology provides an oral care device or component, including an additive-manufactured thermoplastic polyurethane composition derived from (a) a polyisocyanate component comprising an aromatic diisocyanate; (b) a chain extender component; and optionally, (c) a polyol component.

The disclosed technology further provides an oral care device or component in which the chain extender component comprises 1,6-hexanediol (HDO), 1,4-cyclohexane dimethanol (CHDM) or combinations thereof.

The disclosed technology further provides an oral care device or component in which the aromatic diisocyanate includes MDI.

The disclosed technology further provides an oral care device or component in which the additive manufacturing comprises fused deposition modeling or selective laser sintering.

The disclosed technology further provides an oral care device or component in which the thermoplastic polyurethane is biocompatible.

The disclosed technology further provides an oral care device or component further includes a polyol component.

The disclosed technology further provides an oral care device or component in which the molar ratio of chain extender to polyol component is greater than 25.0.

The disclosed technology further provides an oral care device or component in which the polyol component has a number average molecular weight of at least 650.

The disclosed technology further provides an oral care device or component in which the polyol component incudes a polyether polyol, including one or more of PTMEG, an ethylene oxide capped propylene oxide, or combinations thereof.

The disclosed technology further provides an oral care device or component in which the polyol component includes a polyester polyol, including polycaprolactone.

The disclosed technology further provides an oral care device or component in which the chain extender component is from 25 wt% to 35 wt% of the total weight of the composition.

The disclosed technology further provides an oral care device or component in which the polyisocyanate component further includes TDI, IPDI, LDI, BDI, PDI, CHDI, TODI, NDI, HXDI or any combination thereof.

The disclosed technology further provides an oral care device or component in which the chain extender component includes HDO/CHDM and the polyol component includes poly(tetramethylene ether glycol).

The disclosed technology further provides an oral care device or component in which in which the thermoplastic polyurethane further includes one or more of colorants, antioxidants (including phenolics, phosphites, thioesters and/or amines), radio opacifiers, stabilizers, lubricatns, inhibitors, hydrolysis stabilizers, hindered amine light stabilizers, benzotriazole UV absorber, heat stabilizers, stabilizers to prevent discoloration, dyes, pigments, reinforcing agents, or any combination thereof.

The disclosed technology further provides an oral care device or component in which the oral care device or component includes one or more of a dental aligner, a dental retainer or an orthodontic device.

The disclosed technology further provides an oral care device or component in which the oral care device or component is personalized to a patient.

The disclosed technology further provides a method of fabricating a three-dimensional oral care device or component including the steps of (I) operating a system for solid freeform fabrication of an object in which the system includes a solid freeform fabrication apparatus that operates to form a three-dimensional oral care device or component from a building material including a thermoplastic polyurethane derived from (a) a polyisocyanate component including an aromatic diisocyanate; (b) a chain extender component; and (c) an optional polyol component.

The disclosed technology further provides a directly formed oral care device or component including a selectively deposited thermoplastic polyurethane composition derived from (a) an an aromatic diisocyanate; (b) a chain extender component; and (c) an optional polyol component.

The disclosed technology further provides a medical device or component including an additive-manufactured thermoplastic polyurethane composition derived from (a) a polyisocyanate component comprising an aromatic diisocyanate; (b) a chain extender component; and optionally, (c) a polyol component.

The disclosed technology further provides a medical device or component in which the medical device or component includes one or more of a splint, an external support brace, or an orthopedic device.

### DETAILED DESCRIPTION

Various preferred features and embodiments will be described below by way of non-limiting illustration.

The disclosed technology provides thermoplastic polyurethane compositions useful for the direct solid freeform fabrication of oral care and medical devices and components. The described thermoplastic polyurethanes are biocompatible and biodurable, as well as being free from processing aids required by conventional materials used for solid freeform fabrication methods of oral care devices and components.

### The Thermoplastic Polyurethanes.

The thermoplastic polyurethanes useful in the described technology are derived from (a) an aromatic diisocyanate component, (b) a polyol component, and (c) a chain extender component, where the molar ratio of (c) to (b) is greater than 25. The TPU compositions described herein are made using (a) a polyisocyanate component. The polyisocyanate and/or polyisocyanate component includes one or more polyisocyanates. In some embodiments, the polyisocyanate component includes one or more diisocyanates.

In some embodiments, the polyisocyanate and/or polyisocyanate component includes an alpha, omega-alkylene diisocyanate having from 5 to 20 carbon atoms.

In some embodiments, the polyisocyanate component includes one or more aromatic diisocyanates. In some embodiments, the polyisocyanate component is essentially free of, or even completely free of, aliphatic diisocyanates.

Examples of useful polyisocyanates include aromatic diisocyanates such as 4,4'-methylenebis(phenyl isocyanate) (MDI), m-xylene diisocyanate (XDI), phenylene-1,4-diisocyanate, naphthalene-1,5-diisocyanate, and toluene diisocyanate (TDI); as well as aliphatic diisocyanates such as isophorone diisocyanate (IPDI), 1,4-cyclohexyl diisocyanate (CHDI), decane-1,10-diisocyanate, lysine diisocyanate (LDI), 1,4-butane diisocyanate (BDI), isophorone diisocyanate (PDI), 3,3'-dimethyl-4,4'-biphenylene diisocyanate (TODI), 1,5-naphthalene diisocyanate (NDI), and dicyclohexylmethane-4,4'-diisocyanate (H12MDI). Mixtures of two or more polyisocyanates may be used. In some embodiments, the polyisocyanate is MDI.

The TPU compositions described herein are made using (b) a polyol component.

Polyols include polyether polyols, polyester polyols, polycarbonate polyols, polysiloxane polyols, and combinations thereof.

Suitable polyols, which may also be described as hydroxyl terminated intermediates, when present, may include one or more hydroxyl terminated polyesters, one or more hydroxyl terminated polyethers, one or more hydroxyl terminated polycarbonates, one or more hydroxyl terminated polysiloxanes, or mixtures thereof.

Suitable hydroxyl terminated polyester intermediates include linear polyesters having a number average molecular weight (Mn) of from about 500 to about 10,000, from about 700 to about 5,000, or from about 700 to about 4,000, and generally have an acid number less than 1.3 or less than 0.5. The molecular weight is determined by assay of the terminal functional groups and is related to the number average molecular weight. The polyester intermediates may be produced by (1) an esterification reaction of one or more glycols with one or more dicarboxylic acids or anhydrides or (2) by transesterification reaction, i.e., the reaction of one or more glycols with esters of dicarboxylic acids. Mole ratios generally in excess of more than one mole of glycol to acid are preferred so as to obtain linear chains having a preponderance of terminal hydroxyl groups. Suitable polyester intermediates also include various lactones such as polycaprolactone typically made from ε-caprolactone and a bifunctional initiator such as diethylene glycol. The dicarboxylic acids of the desired polyester can be aliphatic, cycloaliphatic, aromatic, or combinations thereof. Suitable dicarboxylic acids which may be used alone or in mixtures generally have a total of from 4 to 15 carbon atoms and include: succinic, glutaric, adipic, pimelic, suberic, azelaic, sebacic, dodecanedioic, isophthalic, terephthalic, cyclohexane dicarboxylic, and the like. Anhydrides of the above dicarboxylic acids such as phthalic anhydride, tetrahydrophthalic anhydride, or the like, can also be used. Adipic acid is a preferred acid. The glycols which are reacted to form a desirable polyester intermediate can be aliphatic, aromatic, or combinations thereof, including any of the glycols described in the chain extender section, and have a total of from 2 to 20 or from 2 to 12 carbon atoms. Suitable examples include ethylene glycol, 1,2-propanediol, 1,3-propanediol, 1,3-butanediol, 1,4-butanediol, 1,5-pentanediol, 1,6-hexanediol, 2,2-dimethyl-1,3-propanediol, 1,4-cyclohexanedimethanol, decamethylene glycol, dodecamethylene glycol, and mixtures thereof.

The polyol component may also include one or more polycaprolactone polyester polyols. The polycaprolactone polyester polyols useful in the technology described herein include polyester diols derived from caprolactone monomers. The polycaprolactone polyester polyols are terminated by primary hydroxyl groups. Suitable polycaprolactone polyester polyols may be made from ε-caprolactone and a bifunctional initiator such as diethylene glycol, 1,4-butanediol, or any of the other glycols and/or diols listed herein. In some embodiments, the polycaprolactone polyester polyols are linear polyester diols derived from caprolactone monomers.

Useful examples include CAPA™ 2202A, a 2000 number average molecular weight (Mn) linear polyester diol, and CAPA™ 2302A, a 3000 Mn linear polyester diol, both of which are commercially available from Perstorp Polyols Inc. These materials may also be described as polymers of 2-oxepanone and 1,4-butanediol.

The polycaprolactone polyester polyols may be prepared from 2-oxepanone and a diol, where the diol may be 1,4-butanediol, diethylene glycol, monoethylene glycol, 1,6-hexanediol, 2,2-dimethyl-1,3-propanediol, or any combination thereof. In some embodiments, the diol used to prepare the polycaprolactone polyester polyol is linear. In some embodiments, the polycaprolactone polyester polyol is prepared from 1,4-butanediol. In some embodiments, the polycaprolactone polyester polyol has a number average molecular weight from 500 to 10,000, or from 500 to 5,000, or from 1,000 or even 2,000, or 2,000 to 4,000 or even 3000.

Suitable hydroxyl terminated polyether intermediates include polyether polyols derived from a diol or polyol having a total of from 2 to 15 carbon atoms, in some embodiments an alkyl diol or glycol which is reacted with an ether comprising an alkylene oxide having from 2 to 6 carbon atoms, typically ethylene oxide or propylene oxide or mixtures thereof. For example, hydroxyl functional polyether can be produced by first reacting propylene glycol with propylene oxide followed by subsequent reaction with ethylene oxide. Primary hydroxyl groups resulting from ethylene oxide are more reactive than secondary hydroxyl groups and thus are preferred. Useful commercial polyether polyols include poly(ethylene glycol) comprising ethylene oxide reacted with ethylene glycol, poly(propylene glycol) comprising propylene oxide reacted with propylene glycol, poly(tetramethylene ether glycol) comprising water reacted with tetrahydrofuran which can also be described as polymerized tetrahydrofuran, and which is commonly referred to as PTMEG. In some embodiments, the polyether intermediate includes PTMEG. Suitable polyether polyols also include polyamide adducts of an alkylene oxide and can include, for example, ethylenediamine adduct comprising the reaction product of ethylenediamine and propylene oxide, diethylenetriamine adduct comprising the reaction product of diethylenetriamine with propylene oxide, and similar polyamide type polyether polyols. Copolyethers can also be utilized in the described compositions. Typical copolyethers include the reaction product of THF and ethylene oxide or THF and propylene oxide. These are available from BASF as PolyTHF® B, a block copolymer, and poly THF® R, a random copolymer. The various polyether intermediates generally have a number average molecular weight (Mn) as determined by assay of the terminal functional groups which is an average molecular weight greater than about 1,000, such as from about 1,000 to about 10,000, from about 1,000 to about 5,000, or from about 1,000 to about 2,500. In some embodiments, the polyether intermediate includes a blend of two or more different molecular weight polyethers, such as a blend of 2,000 Mₙ and 1000 Mₙ PTMEG.

Suitable hydroxyl terminated polycarbonates include those prepared by reacting a glycol with a carbonate. U.S. Patent No. 4,131,731 is hereby incorporated by reference for its disclosure of hydroxyl terminated polycarbonates and their preparation. Such polycarbonates are linear and have terminal hydroxyl groups with essential exclusion of other terminal groups. The essential reactants are glycols and carbonates. Suitable glycols are selected from cycloaliphatic and aliphatic diols containing 4 to 40, and or even 4 to 12 carbon atoms, and from polyoxyalkylene glycols containing 2 to 20 alkoxy groups per molecule with each alkoxy group containing 2 to 4 carbon atoms. Suitable diols include aliphatic diols containing 4 to 12 carbon atoms such as 1,4-butanediol, 1,5-pentanediol, neopentyl glycol, 1,6-hexanediol, 2,2,4-trimethyl-1,6-hexanediol, 1,10-decanediol, hydrogenated dilinoleylglycol, hydrogenated dioleylglycol, 3-methyl-1,5-pentanediol; and cycloaliphatic diols such as 1,3-cyclohexanediol, 1,4-dimethylolcyclohexane, 1,4-cyclohexanediol-, 1,3-dimethylolcyclohexane-, 1,4-endomethylene-2-hydroxy-5-hydroxymethyl cyclohexane, and polyalkylene glycols. The diols used in the reaction may be a single diol or a mixture of diols depending on the properties desired in the finished product. Polycarbonate intermediates which are hydroxyl terminated are generally those known to the art and in the literature. Suitable carbonates are selected from alkylene carbonates composed of a 5 to 7 member ring. Suitable carbonates for use herein include ethylene carbonate, trimethylene carbonate, tetramethylene carbonate, 1,2-propylene carbonate, 1,2-butylene carbonate, 2,3-butylene carbonate, 1,2-ethylene carbonate, 1,3-pentylene carbonate, 1,4-pentylene carbonate, 2,3-pentylene carbonate, and 2,4-pentylene carbonate. Also, suitable herein are dialkylcarbonates, cycloaliphatic carbonates, and diarylcarbonates. The dialkylcarbonates can contain 2 to 5 carbon atoms in each alkyl group and specific examples thereof are diethylcarbonate and dipropylcarbonate. Cycloaliphatic carbonates, especially dicycloaliphatic carbonates, can contain 4 to 7 carbon atoms in each cyclic structure, and there can be one or two of such structures. When one group is cycloaliphatic, the other can be either alkyl or aryl. On the other hand, if one group is aryl, the other can be alkyl or cycloaliphatic. Examples of suitable diarylcarbonates, which can contain 6 to 20 carbon atoms in each aryl group, are diphenylcarbonate, ditolylcarbonate, and dinaphthylcarbonate.

Suitable polysiloxane polyols include alpha-omega-hydroxyl or amine or carboxylic acid or thiol or epoxy terminated polysiloxanes. Examples include poly(dimethysiloxane) terminated with a hydroxyl or amine or carboxylic acid or thiol or epoxy group. In some embodiments, the polysiloxane polyols are hydroxyl terminated polysiloxanes. In some embodiments, the polysiloxane polyols have a number-average molecular weight in the range from 300 to 5,000, or from 400 to 3,000.

Polysiloxane polyols may be obtained by the dehydrogenation reaction between a polysiloxane hydride and an aliphatic polyhydric alcohol or polyoxyalkylene alcohol to introduce the alcoholic hydroxy groups onto the polysiloxane backbone.

In some embodiments, the polysiloxanes may be represented by one or more compounds having the following formula: 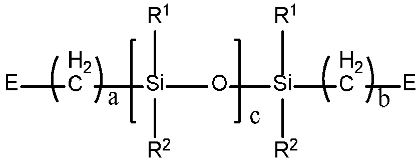 in which: each R¹ and R² are independently a 1 to 4 carbon atom alkyl group, a benzyl, or a phenyl group; each E is OH or NHR³ where R³ is hydrogen, a 1 to 6 carbon atoms alkyl group, or a 5 to 8 carbon atoms cyclo-alkyl group; a and b are each independently an integer from 2 to 8; c is an integer from 3 to 50. In amino-containing polysiloxanes, at least one of the E groups is NHR³. In the hydroxyl-containing polysiloxanes, at least one of the E groups is OH. In some embodiments, both R¹ and R² are methyl groups.

Suitable examples include alpha-omega-hydroxypropyl terminated poly(dimethysiloxane) and alpha-omega-amino propyl terminated poly(dimethysiloxane), both of which are commercially available materials. Further examples include copolymers of the poly(dimethysiloxane) materials with a poly(alkylene oxide).

The polyol component may include poly(ethylene glycol), poly(tetramethylene ether glycol), poly(trimethylene oxide), ethylene oxide capped poly(propylene glycol), poly(butylene adipate), poly(ethylene adipate), poly(hexamethylene adipate), poly(tetramethylene-co-hexamethylene adipate), poly(3-methyl-1,5-pentamethylene adipate), polycaprolactone diol, poly(hexamethylene carbonate) glycol, poly(pentamethylene carbonate) glycol, poly(trimethylene carbonate) glycol, dimer fatty acid based polyester polyols, vegetable oil based polyols, or any combination thereof.

Examples of dimer fatty acids that may be used to prepare suitable polyester polyols include Priplast™ polyester glycols/polyols commercially available from Croda and Radia® polyester glycols commercially available from Oleon.

In some embodiments, the polyol component includes a polyether polyol, a polycarbonate polyol, a polycaprolactone polyol, or any combination thereof.

In some embodiments, the polyol component includes a polyether polyol. In some embodiments, the polyol component is essentially free of or even completely free of polyether polyols. In some embodiments, the polyol component used to prepare the TPU is substantially free of, or even completely free of polysiloxanes.

In some embodiments, the polyol component includes polycaprolactone, poly(tetramethylene ether glycol), or a trifunctional ether, such as an end-capped polypropylene oxide, and the like, or combinations thereof. In some embodiments, the polyol component includes polycaprolactone. In some embodiments, the polyol component includes an end-capped polypropylene oxide. In some embodiments, the polyol component includes poly(tetramethylene ether glycol).

In some embodiments, the polyol has a number average molecular weight of at least 650. In other embodiments, the polyol has a number average molecular weight of at least 2000, 2,500, 3,000, and/or a number average molecular weight up to 2,500, 3,000, 4000, or even 6,000.

The TPU compositions described herein are made using c) a chain extender component. Chain extenders include aromatic glycols, diols, diamines, and combination thereof.

Suitable chain extenders include relatively small polyhydroxy compounds, for example lower aliphatic or short chain glycols having from 2 to 20, or 2 to 12, or 2 to 10 carbon atoms. Suitable examples include ethylene glycol, diethylene glycol, propylene glycol, dipropylene glycol (DPG), 1,4-butanediol (BDO), 1,6-hexanediol (HDO), 1,3-butanediol, 1,5-pentanediol, neopentylglycol, 1,4-cyclohexanedimethanol (CHDM), 2,2-bis[4-(2-hydroxyethoxy) phenyl]propane (HEPP), hexamethylenediol, heptanediol, nonanediol, dodecanediol, 3-methyl-1,5-pentanediol, ethylenediamine, butanediamine, hexamethylenediamine, and hydroxyethyl resorcinol (HER), and the like, as well as mixtures thereof. In some embodiments the chain extender includes HDO. In some embodiments the chain extender includes HDO and CHDM.

In some embodiments, the mole ratio of the chain extender to the polyol is greater than 25. In other embodiments in which the thermoplastic polyurethane is free from a polyol component, the mole ratio of the chain extender to the polyol is infinite.

The thermoplastic polyurethanes described herein may also be considered to be thermoplastic polyurethane (TPU) compositions. In such embodiments, the compositions may contain one or more TPU. These TPU are prepared by reacting: a) the polyisocyanate component described above; b) the polyol component described above; and c) the chain extender component described above, where the reaction may be carried out in the presence of a catalyst. At least one of the TPU in the composition must meet the parameters described above making it suitable for solid freeform fabrication, and in particular fused deposition modeling.

The means by which the reaction is carried out is not overly limited, and includes both batch and continuous processing. In some embodiments, the technology deals with batch processing of aromatic TPU. In some embodiments, the technology deals with continuous processing of aromatic TPU.

The described compositions include the TPU materials described above and also TPU compositions that include such TPU materials and one or more additional components. These additional components include other polymeric materials that may be blended with the TPU described herein. These additional components include one or more additives that may be added to the TPU, or blend containing the TPU, to impact the properties of the composition.

The TPU described herein may also be blended with one or more other polymers. The polymers with which the TPU described herein may be blended are not overly limited. In some embodiments, the described compositions include two or more of the described TPU materials. In some embodiments, the compositions include at least one of the described TPU materials and at least one other polymer, which is not one of the described TPU materials.

Polymers that may be used in combination with the TPU materials described herein also include more conventional TPU materials such as non-caprolactone polyester-based TPU, polyether-based TPU, or TPU containing both non-caprolactone polyester and polyether groups. Other suitable materials that may be blended with the TPU materials described herein include polycarbonates, polyolefins, styrenic polymers, acrylic polymers, polyoxymethylene polymers, polyamides, polyphenylene oxides, polyphenylene sulfides, polyvinylchlorides, chlorinated polyvinylchlorides, polylactic acids, or combinations thereof.

Polymers for use in the blends described herein include homopolymers and copolymers. Suitable examples include: (i) a polyolefin (PO), such as polyethylene (PE), polypropylene (PP), polybutene, ethylene propylene rubber (EPR), polyoxyethylene (POE), cyclic olefin copolymer (COC), or combinations thereof; (ii) a styrenic, such as polystyrene (PS), acrylonitrile butadiene styrene (ABS), styrene acrylonitrile (SAN), styrene butadiene rubber (SBR or HIPS), polyalphamethylstyrene, styrene maleic anhydride (SMA), styrene-butadiene copolymer (SBC) (such as styrene-butadiene-styrene copolymer (SBS) and styrene-ethylene/butadiene-styrene copolymer (SEBS)), styrene-ethylene/propylene-styrene copolymer (SEPS), styrene butadiene latex (SBL), SAN modified with ethylene propylene diene monomer (EPDM) and/or acrylic elastomers (for example, PS-SBR copolymers), or combinations thereof; (iii) a thermoplastic polyurethane (TPU) other than those described above; (iv) a polyamide, such as Nylon™, including polyamide 6,6 (PA66), polyamide 1,1 (PA11), polyamide 1,2 (PA12), a copolyamide (COPA), or combinations thereof; (v) an acrylic polymer, such as polymethyl acrylate, polymethylmethacrylate, a methyl methacrylate styrene (MS) copolymer, or combinations thereof; (vi) a polyvinylchloride (PVC), a chlorinated polyvinylchloride (CPVC), or combinations thereof; (vii) a polyoxyemethylene, such as polyacetal; (viii) a polyester, such as polyethylene terephthalate (PET), polybutylene terephthalate (PBT), copolyesters and/or polyester elastomers (COPE) including polyether-ester block copolymers such as glycol modified polyethylene terephthalate (PETG), polylactic acid (PLA), polyglycolic acid (PGA), copolymers of PLA and PGA, or combinations thereof; (ix) a polycarbonate (PC), a polyphenylene sulfide (PPS), a polyphenylene oxide (PPO), or combinations thereof; or combinations thereof.

In some embodiments, these blends include one or more additional polymeric materials selected from groups (i), (iii), (vii), (viii), or some combination thereof. In some embodiments, these blends include one or more additional polymeric materials selected from group (i). In some embodiments, these blends include one or more additional polymeric materials selected from group (iii). In some embodiments, these blends include one or more additional polymeric materials selected from group (vii). In some embodiments, these blends include one or more additional polymeric materials selected from group (viii).

The additional optional additives suitable for use in the TPU compositions described herein are not overly limited. Suitable additives include pigments, UV stabilizers, UV absorbers, antioxidants, lubricity agents, heat stabilizers, hydrolysis stabilizers, cross-linking activators, biocompatible flame retardants, layered silicates, colorants, reinforcing agents, adhesion mediators, impact strength modifiers, antimicrobials, radio opacifiers, fillers and any combination thereof. It is to be noted that the TPU compositions of the invention disclosed herein do not require the use of inorganic, organic or inert fillers, such as are talc, calcium carbonate, TiO2, powders which, while not wishing to be bound by theory, it is believed may assist in printability of the TPU composition. Thus, in some embodiments, the disclosed technology may include a filler, and in some embodiments, the disclosed technology may be free of fillers.

The TPU compositions described herein may also include additional additives, which may be referred to as a stabilizer. The stabilizers may include antioxidants such as phenolics, phosphites, thioesters, and amines, light stabilizers such as hindered amine light stabilizers and benzothiazole UV absorbers, and other process stabilizers and combinations thereof. In one embodiment, the preferred stabilizer is Irganox 1010 from BASF and Naugard 445 from Chemtura. The stabilizer is used in the amount from about 0.1 weight percent to about 5 weight percent, in another embodiment from about 0.1 weight percent to about 3 weight percent, and in another embodiment from about 0.5 weight percent to about 1.5 weight percent of the TPU composition.

Still further optional additives may be used in the TPU compositions described herein. The additives include colorants, antioxidants (including phenolics, phosphites, thioesters, and/or amines), stabilizers, lubricants, inhibitors, hydrolysis stabilizers, light stabilizers, hindered amines light stabilizers, benzotriazole UV absorber, heat stabilizers, stabilizers to prevent discoloration, dyes, pigments, reinforcing agents and combinations thereof.

All of the additives described above may be used in an effective amount customary for these substances. The non-flame retardants additives may be used in amounts of from about 0 to about 30 weight percent, in one embodiment from about 0.1 to about 25 weight percent, and in another embodiment about 0.1 to about 20 weight percent of the total weight of the TPU composition.

These additional additives can be incorporated into the components of, or into the reaction mixture for, the preparation of the TPU resin, or after making the TPU resin. In another process, all the materials can be mixed with the TPU resin and then melted or they can be incorporated directly into the melt of the TPU resin.

The TPU materials described above may be prepared by a process that includes the step of (I) reacting: a) the aromatic diisocyanate component described above; b) the polyol component described above; and c) the chain extender component described above, where the reaction may be carried out in the presence of a catalyst, resulting in a thermoplastic polyurethane composition.

The process may further include the step of: (II) mixing the TPU composition of step (I) with one or more blend components, including one or more additional TPU materials and/or polymers, including any of those described above.

The process may further include the step of: (II) mixing the TPU composition of step (I) with one or more of the additional additives described above.

The process may further include the step of: (II) mixing the TPU composition of step (I) with one or more blend components, including one or more additional TPU materials and/or polymers, including any of those described above, and/or the step of: (III) mixing the TPU composition of step (I) with one or more of the additional additives described above.

### The Systems and Methods.

The solid freeform fabrication systems and the methods of using the same useful in the described technology are not overly limited. It is noted that the described technology provides certain thermoplastic polyurethanes that are better suited for the solid freeform fabrication of medical devices and components, than current materials and other thermoplastic polyurethanes. It is noted that some solid freeform fabrication systems, including some fused deposition modeling systems may be better suited for processing certain materials, including thermoplastic polyurethanes, due to their equipment configurations, processing parameters, etc. However, the described technology is not focused on the details of solid freeform fabrication systems, including some fused deposition modeling systems, rather the described technology is focused on providing certain thermoplastic polyurethanes that are better suited for solid freeform fabrication of medical devices and components.

The extrusion-type additive manufacturing systems and processes useful in the present invention include systems and processes that build parts layer-by-layer by heating the building material to a semi-liquid state and extruding it according to computer-controlled paths. The material, supplied as a strand or resin, may be dispensed as a semi-continuous flow and/or filament of material from the dispenser or it may alternatively be dispensed as individual droplets. FDM often uses two materials to complete a build. A modeling material is used to constitute the finished piece. A support material may also be used to act as scaffolding for the modeling material. The building material, e.g., TPU, is fed from the systems material stores to its print head, which typically moves in a two dimensional plane, depositing material to complete each layer before the base moves along a third axis to a new level and/or plane and the next layer begins. Once the system is done building, the user may remove the support material away or even dissolve it, leaving a part that is ready to use. In some embodiments, the additive manufacturing systems and processes will include a support material which includes a TPU different from the inventive TPU disclosed herein. In some embodiments, the systems and processes are free of the support material.

The powder or granular type of additive manufacturing systems and processes useful in the present invention SLS involves the use of a high power laser (for example, a carbon dioxide laser to fuse small particles of the material, e.g. TPU, into a mass that has a desired three-dimensional shape. Production by selective fusion of layers is a method for producing articles that consists in depositing layers of materials in powder form, selectively melting a portion or a region of a layer, depositing a new layer of powder and again melting a portion of said layer, and continuing in this manner until the desired object is obtained. The selectivity of the portion of the layer to be melted is obtained for example by using absorbers, inhibitors, masks, or via the input of focused energy, such as a laser or electromagnetic beam, for example. Sintering by the addition of layers is preferred, in particular rapid prototyping by sintering using a laser. Rapid prototyping is a method used to obtain parts of complex shape without tools and without machining, from a three-dimensional image of the article to be produced, by sintering superimposed powder layers using a laser. General information about rapid prototyping by laser sintering is provided in U.S. Pat. No. 6,136,948 and applications WO96/06881 and US20040138363.

Machines for implementing these methods may comprise a construction chamber on a production piston, surrounded on the left and right by two pistons feeding the powder, a laser, and means for spreading the powder, such as a roller. The chamber is generally maintained at constant temperature to avoid deformations.

Other production methods by layer additions' such as those described in WO 01/38061 and EP1015214 are also suitable. These two methods use infrared heating to melt the powder. The selectivity of the molten parts is obtained in the case of the first method by the use of inhibitors, and in the case of the second method by the use of a mask. Another method is described in application DE10311438. In this method, the energy for melting the polymer is supplied by a microwave generator and selectivity is obtained by using a susceptor.

The disclosed technology further provides the use of the described thermoplastic polyurethanes in the described systems and methods, and the medical devices and components made from the same.

### The Medical Devices, Components and Applications.

The processes described herein may utilize the thermoplastic polyurethanes described herein to produce various medical devices and components.

As with all additive manufacturing there is particular value for such technology in making articles as part of rapid prototyping and new product development, as part of making custom and/or one time only parts, or similar applications where mass production of an article in large numbers is not warranted and/or practical.

Useful oral care devices and components which may be additive-manufactured from the compositions of the invention include dental aligners, retainers, orthodontic devices, and the like. Useful medical devices and component which may be additive-manufactured from the compositions of the invention include splints, external support braces, orthopedic devices, and the like.

The amount of each chemical component described is presented exclusive of any solvent or diluent oil, which may be customarily present in the commercial material, that is, on an active chemical basis, unless otherwise indicated. However, unless otherwise indicated, each chemical or composition referred to herein should be interpreted as being a commercial grade material which may contain the isomers, byproducts, derivatives, and other such materials which are normally understood to be present in the commercial grade.

It is known that some of the materials described above may interact in the final formulation, so that the components of the final formulation may be different from those that are initially added. For instance, metal ions (of, e.g., a flame retardant) can migrate to other acidic or anionic sites of other molecules. The products formed thereby, including the products formed upon employing the composition of the technology described herein in its intended use, may not be susceptible of easy description. Nevertheless, all such modifications and reaction products are included within the scope of the technology described herein; the technology described herein encompasses the composition prepared by admixing the components described above.

### EXAMPLES

The technology described herein may be better understood with reference to the following non-limiting examples.

### Example 1

*Materials.* Several thermoplastic polyurethanes (TPU) are prepared and evaluated for their suitability of use in direct solid free form fabrication of an oral care device. Inventive TPU-A is a TPU containing a polyether polyol with a molar ratio of chain extender to polyol of about 31.3. Inventive TPU-B is a TPU containing an ethylene oxide-capped propylene oxide polyol with a molar ratio of chain extender to polyol of about 178. Inventive TPU-C is a TPU containing a polyester polyol with a molar ratio of chain extender to polyol of about 29.7. Inventive TPU-D is a TPU containing no polyol, and thus having an infinite molar ratio of polyol to chain extender.

Each TPU material is tested to determine its suitability for use in select freeform fabrication processes. Each TPU material is extruded from resin into approximately 1.8mm diameter rods using s single screw extruder. Tensile bars are printed utilizing a fused deposition modeling process on a MakerBot 2X desktop 3D printer running MakerBot Desktop Software Version 3.7 with the following test parameters:

| | |
|---|---|
| Extrusion Temperature | 200°C-230°C |
| Build Platform Temperature | 40°C-150°C |
| Print Speed | 30 mm/s - 120 mm/s |

Results of this testing are summarized below in Table 1.

**Table 1**

| | TPU-A | TPU-B | TPU-C | TPU-D |
|---|---|---|---|---|
| Chain Extender:Polyol mole ratio | 31.3 | 178 | 29.7 | ∞ |
| Print Speed (mm/sec) | 90 | 90 | 90 | 90 |

As illustrated by the results, the inventive TPU compositions provide compositions which are suitable for solid freeform fabrication.

### Example 2

A 3D-printed sample of inventive TPU composition D was evaluated for tensile strength and elongation on the "x", "y" and "z" axes (identified per ASTM 52921) pursuant to ASTM D412 in comparison with a standard injection-molded TPU material of the same composition. Results are shown in Table 2 below:

**Table 2**

| | Tensile Strength (psi) |
|---|---|
| Standard Injection Molded TPU "D" | 7021 ± 128 |
| TPU-D xy axis | 5951 ± 226 |
| TPU-D xz axis | 8117 ± 538 |
| TPU-D zx axis | 1133 ±519 |

As can be seen in Table 2, the ultimate tensile strength of the inventive 3D printed TPU material is comparable to that of an injection molded material when printed using the xz axis direction.

Molecular weight distributions can be measured on the Waters gel permeation chromatograph (GPC) equipped with Waters Model 515 Pump, Waters Model 717 autosampler and Waters Model 2414 refractive index detector held at 40°C. The GPC conditions may be a temperature of 40°C, a column set of Phenogel Guard + 2x mixed D (5u), 300 x 7.5 mm, a mobile phase of tetrahydrofuran (THF) stabilized with 250 ppm butylated hydroxytoluene, a flow rate of 1.0 ml/min, an injection volume of 50 µl, sample concentration ∼0.12%, and data acquisition using Waters Empower Pro Software. Typically a small amount, typically approximately 0.05 gram of polymer, is dissolved in 20 ml of stabilized HPLC-grade THF, filtered through a 0.45-micron polytetrafluoroethylene disposable filter (Whatman), and injected into the GPC. The molecular weight calibration curve may be established with EasiCal® polystyrene standards from Polymer Laboratories.

Each of the documents referred to above is incorporated herein by reference, including any prior applications, whether or not specifically listed above, from which priority is claimed. The mention of any document is not an admission that such document qualifies as prior art or constitutes the general knowledge of the skilled person in any jurisdiction. Except in the Examples, or where otherwise explicitly indicated, all numerical quantities in this description specifying amounts of materials, reaction conditions, molecular weights, number of carbon atoms, and the like, are to be understood as modified by the word "about." It is to be understood that the upper and lower amount, range, and ratio limits set forth herein may be independently combined. Similarly, the ranges and amounts for each element of the technology described herein can be used together with ranges or amounts for any of the other elements.

As used herein, the transitional term "comprising," which is synonymous with "including," "containing," or "characterized by," is inclusive or open-ended and does not exclude additional, un-recited elements or method steps. However, in each recitation of "comprising" herein, it is intended that the term also encompass, as alternative embodiments, the phrases "consisting essentially of' and "consisting of," where "consisting of' excludes any element or step not specified and "consisting essentially of' permits the inclusion of additional un-recited elements or steps that do not materially affect the basic and novel characteristics of the composition or method under consideration. That is "consisting essentially of' permits the inclusion of substances that do not materially affect the basic and novel characteristics of the composition under consideration.

While certain representative embodiments and details have been shown for the purpose of illustrating the subject technology described herein, it will be apparent to those skilled in this art that various changes and modifications can be made therein without departing from the scope of the subject invention. In this regard, the scope of the technology described herein is to be limited only by the following claims.

The invention is further described by the following numbered clauses.
Clause 1. An oral care device or component, comprising:
   an additive-manufactured thermoplastic polyurethane composition derived from (a) a polyisocyanate component comprising an aromatic diisocyanate; (b) a chain extender component; and optionally, (c) a polyol component.
Clause 2. The oral care device or component of clause 1, wherein the chain extender component comprises 1,6-hexanediol (HDO), 1,4-cyclohexane dimethanol (CHDM) or combinations thereof.
Clause 3. The oral care device or component clause 1, wherein the aromatic diisocyanate comprises MDI.
Clause 4. The oral care device or component of clause 1, wherein the additive manufacturing comprises fused deposition modeling or selective laser sintering.
Clause 5. The oral care device or component of clause 1, wherein the thermoplastic polyurethane is biocompatible.
Clause 6. The oral care device or component of any of clause 1, further comprising a polyol component.
Clause 7. The oral care device or component of clause 6, wherein the molar ratio of chain extender to polyol component is greater than 25.0.
Clause 8. The oral care device or component of clause 7, wherein the polyol component has a number average molecular weight of at least 650.
Clause 9. The oral care device or component of clause 7, wherein the polyol component comprises a polyether polyol comprising one or more of PTMEG, an ethylene oxide capped propylene oxide, or combinations thereof.
Clause 10. The oral care device or component of clause 7, wherein the polyol component comprises a polyester polyol comprising polycaprolactone.
Clause 11. The oral care device or component of clause 1, wherein the chain extender component is from 25 wt% to 35 wt% of the total weight of the composition.
Clause 12. The oral care device or component of clause 1, wherein the polyisocyanate component further comprise TDI, IPDI, LDI, BDI, PDI, CHDI, TODI, NDI, HXDI or any combination thereof.
Clause 13. The oral care device or component of clause 7, wherein the chain extender component comprises HDO/CHDM and the polyol component comprises poly(tetramethylene ether glycol).
Clause 14. The oral care device or component of clause 1, wherein the thermoplastic polyurethane further comprises one or more colorants, antioxidants (including phenolics, phosphites, thioesters, and/or amines), radio opacifiers, stabilizers, lubricants, inhibitors, hydrolysis stabilizers, light stabilizers, hindered amine light stabilizers, benzotriazole UV absorber, heat stabilizers, stabilizers to prevent discoloration, dyes, pigments, reinforcing agents, or any combinations thereof.
Clause 15. The oral care device or component of clause 1, wherein the oral care device or component comprises one or more of a dental aligner, a dental retainer or an orthodontic device.
Clause 16. The oral care device or component of clause 15, wherein the oral care device or component is personalized to a patient.
Clause 17. A method of directly fabricating a three-dimensional oral care device or component, comprising the step of: (I) operating a system for solid freeform fabrication of an object;
   wherein said system comprises a solid freeform fabrication apparatus that operates to form a three-dimensional or care or medical device or component from a building material comprising a thermoplastic polyurethane derived from (a) a polyisocyanate component comprising an aromatic diisocyanate, (b) chain extender component, and (c) an optional polyol component.
Clause 18. A directly formed oral care device or component, comprising:
   a selectively deposited thermoplastic polyurethane composition derived from (a) an aromatic diisocyanate component, (b) a chain extender component, and (c) an optional a polyether or polyester polyol component.
Clause 19. A medical device or component, comprising:
   an additive-manufactured thermoplastic polyurethane composition derived from (a) a polyisocyanate component comprising an aromatic diisocyanate; (b) a chain extender component; and optionally, (c) a polyol component.
Clause 20. The medical device or component of clause 19, wherein the medical device or component comprises one or more of a splint, an external support brace, or an orthopedic device.

## Claims

1. An medical device or component, comprising:
an additive-manufactured thermoplastic polyurethane composition derived from (a) a polyisocyanate component comprising an aromatic diisocyanate; (b) a chain extender component; and, (c) a polyol component, wherein the molar ratio of chain extender to polyol component is greater than 25.0.

2. The medical device or component of claim 1, wherein the chain extender component comprises 1,6-hexanediol (HDO), 1,4-cyclohexane dimethanol (CHDM) or combinations thereof.

3. The medical device or component claim 1, wherein the aromatic diisocyanate comprises MDI.

4. The medical device or component of claim 1, wherein the additive manufacturing comprises fused deposition modeling or selective laser sintering.

5. The medical device or component of claim 1, wherein the polyol component has a number average molecular weight of at least 650.

6. The medical device or component of claim 1, wherein the polyol component comprises a polyether polyol comprising one or more of PTMEG, an ethylene oxide capped propylene oxide, or combinations thereof.

7. The medical device or component of claim 1, wherein the polyol component comprises a polyester polyol comprising polycaprolactone.

8. The medical device or component of claim 1, wherein the chain extender component is from 25 wt% to 35 wt% of the total weight of the composition.

9. The medical device or component of claim 1, wherein the polyisocyanate component further comprise TDI, IPDI, LDI, BDI, PDI, CHDI, TODI, NDI, HXDI or any combination thereof.

10. The medical device or component of claim 1, wherein the chain extender component comprises HDO/CHDM and the polyol component comprises poly(tetramethylene ether glycol).

11. The medical device or component of claim 1, wherein the thermoplastic polyurethane further comprises one or more colorants, antioxidants (including phenolics, phosphites, thioesters, and/or amines), radio opacifiers, stabilizers, lubricants, inhibitors, hydrolysis stabilizers, light stabilizers, hindered amine light stabilizers, benzotriazole UV absorber, heat stabilizers, stabilizers to prevent discoloration, dyes, pigments, reinforcing agents, or any combinations thereof.

12. The medical device or component of claim 1, wherein the medical device or component is an oral care device or component, and, preferably, wherein the oral care device or component comprises one or more of a dental aligner, a dental retainer or an orthodontic device, which is preferably personalized to a patient

13. The medical device or component of any one of claims 1 to 11, wherein the medical device or component comprises one or more of a splint, an external support brace, or an orthopedic device.

14. The medical device or component of claim 12, wherein the oral care device or component is a directly formed oral care device or component, comprising:
a selectively deposited thermoplastic polyurethane composition derived from (a) an aromatic diisocyanate component, (b) a chain extender component, and (c) a polyether or polyester polyol component.

15. A method of directly fabricating a three-dimensional oral care device or component according to claim 12 or claim 14, comprising the step of: (I) operating a system for solid freeform fabrication of an object;
wherein said system comprises a solid freeform fabrication apparatus that operates to form a three-dimensional or care or medical device or component from a building material comprising a thermoplastic polyurethane derived from (a) a polyisocyanate component comprising an aromatic diisocyanate, (b) chain extender component, and (c) a polyol component.
